# EUROPEAN PATENT APPLICATION

(11) **EP 1 541 556 A1**
(43) Date of publication of application: **15.06.2005**
(21) Application number: 03784524.5
(22) Date of filing: 05.08.2003
(51) Int. Cl.: C07D 209/60, C09K 3/00, G03G 9/08

(54) **INDOLENINE COMPOUND, NEAR INFRARED ABSORBER, AND TONER CONTAINING THE SAME**

(30) Priority: 09.08.2002 JP 2002232715
(71) Applicant: Nippon Kayaku Kabushiki Kaisha, Tokyo 102-8172 (JP)
(72) Inventor: TORINIWA, Toshitaka, Saitama-shi, Saitama 338-0001 (JP); YAMAMURA, Shigeo, Saitama-shi, Saitama 331-0812 (JP)
(74) Representative: Gille Hrabal Struck Neidlein Prop Roos
(86) International application number: PCT/JP2003/009938
(87) International publication number: WO 2004/014857

(57) **Abstract**

A novel indolenine compound which has a high molar extinction coefficient (weight extinction coefficient) and shows considerably reduced absorption in the visible light region; an infrared absorber; and a toner containing the absorber, specifically a toner for noncontact thermal fixing, especially such as the flash fixing type, which has satisfactory fixability, is reduced in influence on the color tones of color toners, and has a high molar extinction coefficient. The indolenine compound is represented by the following general formula (1). Also provided are: a near infrared absorber comprising the indolenine compound; and a toner for copiers of the noncontact thermal fixing type, which contains the indolenine compound. 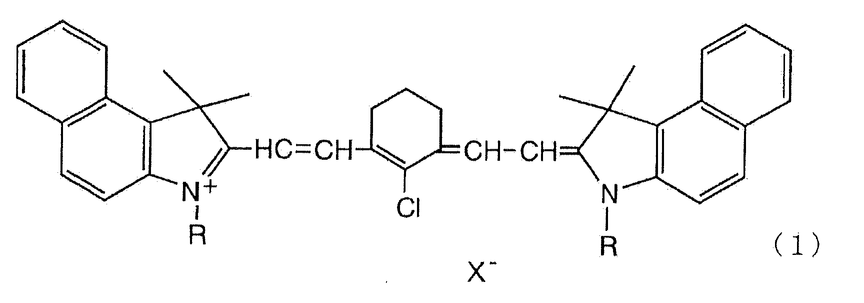 In the formula (1), R represents a group represented by the following general formula (2).

-((CH₂)_{b}O)ₐ(CH₂)_{c}OR₁ (2)

(wherein a is 1 or 0, b and c each independently is 1 or 2, and R₁ represents C₄ or lower alkyl) ; and X⁻ represents an ion for charge neutralization.

## Description

### Technical Field

The present invention relates to an indolenine compound and its use. In particular, the invention relates to a novel indolenine compound, a near infrared absorber and a toner containing the same. More particularly, the invention relates to a novel indolenine compound having a high molar extinction coefficient (weight extinction coefficient) and extremely low absorption in the visible region, an infrared absorber, and a toner containing the same, especially a toner for noncontact thermal fixing, such as flash fixing type.

### Background Art

Conventionally, a heat roll method has been mainly used as a method for fixing an image on a printing medium in an electrophotography method in a copy machine, a printer or the like. However, in this method, a printing medium such as paper having an image formed with a toner is passed between heat rollers for thermally compressing the toner on the printing medium, and thus this method has problems in that clogging occurs in a fixingpart, the resolution is deteriorated because the image is flatten out, and the type of the printing medium is limited, etc.

In contrast, noncontact thermal fixing methods, such as an oven fixing method and a flash fixing method, are proposed as one kind of image fixing methods which do not have the above problems for heat roll method. In such noncontact thermal fixing method, a toner supplied to a printing medium is irradiated with heat or near infrared, and thereby the toner is heated and fixed on a recording medium. A coloring agent contained in a conventional color toner has an absorption in the visible region, but almost no absorption of light in the near infrared region of a discharge tube used for flash fixing, resulting in inferior conversion efficiency to thermal energy and insufficient fixing. In addition, a black color material such as carbon black, which is a coloring agent contained in a black toner, has absorptivity in the near infrared region. However, it does not have sufficient conversion efficiency to thermal energy and hence it has various problems. In particular, to obtain a full color image by superposing a plurality of different color toners, a large amount of color toners is to be supplied to a printing medium. It was very difficult to fix thus supplied color toners on the printing medium substantially.

As a method for solving these problems, it has been proposed in Japanese Patent Application Laying Open (KOKAI) No. 63-161460, Japanese Patent Publication (KOKOKU) No. 7-23965, Japanese Patent Application Laying Open (KOKAI) No. 2000-214626, and Japanese Patent Application Laying Open (KOKAI) No. 2000-214628 that a near infrared absorber such as phthalocyanine dye, aminium dye, and nickel-dithiol complex dye can be dispersed and blended in a toner for fixing the toner with a flash of a discharge tube, for example, a xenon flash lamp.

The molar extinction coefficients of these near infrared absorbers are not so high in the near infrared region. Thus, in order to adequately fuse a binder resin by heat generation of the near infrared absorbers, the amount of the absorbers to be added inevitably increases, resulting in less efficiency and economical disadvantage. Further, increased amount to be added gives impacts on the color tones of toners. Furthermore, in the case of a near infrared absorber having a decomposition temperature of less than 200°C, the absorber is thermally decomposed during melting and kneading to produce a toner. Therefore, decomposition products cause adverse effects on the color tones of the toner in addition to the decrease of near infrared absorptivity. There has been a demand for a near infrared absorber having a decomposition temperature higher than 200°C, a high molar extinction coefficient (and weight extinction coefficient), and reduced absorption in the visible region.

To satisfy this demand, it was proposed in Japanese Patent Application Laying Open (KOKAI) No. 2002-156779 that a toner is efficiently fixed on a noncontact thermal fixing apparatus by fixing a near infrared absorber on the surface of a toner particle. However, there has been a problem that sufficient effects cannot be obtained only by using a well-known near infrared absorber.

### Problems to be Solved by the Invention

The present invention has been made in light of such circumstances and an object of the present invention relates to a novel indolenine compound having a high molar extinction coefficient (and weight extinction coefficient) and exhibiting considerably reduced absorption in the visible light region; an infrared absorber and a toner containing the same, and more particularly to a toner for noncontact thermal fixing such as flash fixing type, which has excellent fixability, a reduced influence on the color tones of color toners, and a high molar extinction coefficient.

### Disclosure of the Invention

The present inventors have made intensive efforts to solve the problems described above. As a result, the inventors have found that the problems can be solved by using a near-infrared absorber represented by the following general formula (1) as a near-infrared absorber for an electrophotographic toner, thereby completing the present invention.

Namely, the present invention relates to the following aspects (1) to (7):
(1) An indolenine compound represented by the following general formula (1): 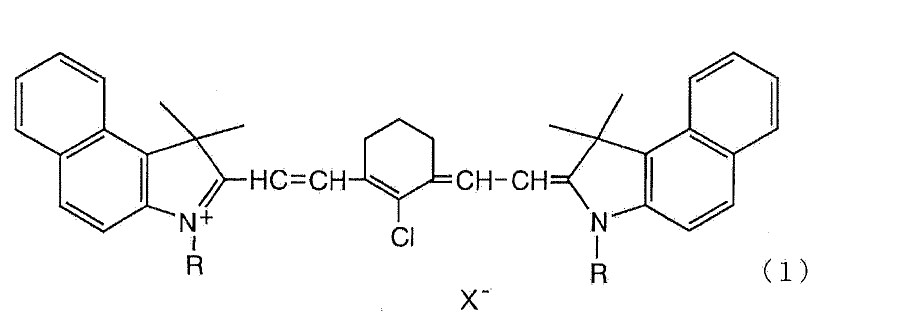 wherein R represents a group represented by the following general formula (2):

   - ((CH₂)_{b}O)ₐ(CH₂)_{c}OR₁ (2)

   wherein a, b and c each represent an integer: a is 1 or 0; b and c each independently represent 1 or 2; and R₁ represents an alkyl group having 4 or less carbon atoms; and
   X⁻ represents BF₄⁻, PF₆⁻, I⁻ or NO₃⁻.
(2) The indolenine compound described in (1), wherein R represents the following general formula (3):

   -(CH₂)_{c}OR₁ (3)

   wherein c represents 1 or 2, and R₁ represents an alkyl group having 4 or less carbon atoms.
(3) An infrared absorber containing as an active ingredient the indolenine compound described in (1) or (2).
(4) A toner containing an indolenine compound represented by the following general formula (4): 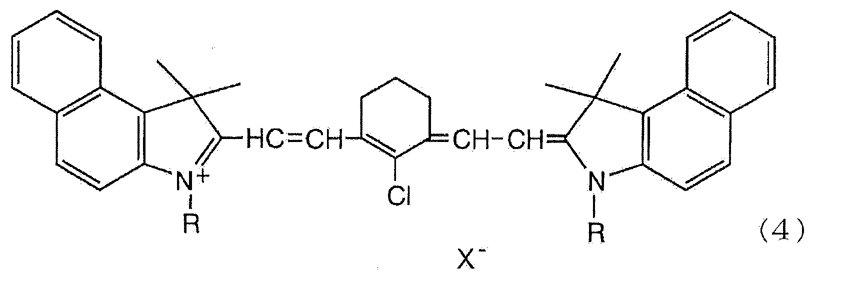 wherein R represents a group represented by the following general formula (5):

   - ((CH₂)_{b}O)ₐ(CH₂)_{c}OR₁ (5)

   wherein a, b and c each represent an integer: a is 1 or 0; b and c each independently represent 1 or 2; and R₁ represents an alkyl group having 4 or less carbon atoms; and
   X⁻ represents an ion for neutralization of an electric charge.
(5) The toner described in (4), wherein R represents the following general formula (6):

   -(CH₂)_{c}OR₁ (6)

   wherein c represents 1 or 2, and R₁ represents an alkyl group having 4 or less carbon atoms.
(6) The toner described in (4) or (5), wherein X⁻ represents BF₄⁻, PF₆⁻, I⁻, NO₃⁻ or SbF₆⁻.

### Embodiments of the Invention

The present invention will be described in detail.

In the formula (4), R represents the formula (5), preferably the formula (6).

"a" and "b" of the formula (5) and "c" of the formulas (5) and (6) each represent an integer, and a is 1 or 0; b and c each independently represent 1 or 2; and R₁ represents an alkyl group having 4 or less carbon atoms.

Specific examples of R include alkoxyalkyl groups such as a methoxymethyl group, a methoxyethyl group, an ethoxyethyl group, and a butoxyethyl group; and alkoxyalkoxyalkyl groups such as a methoxymethoxymethoxy group and an ethoxyethoxyethoxy group. Preferable are, for example, a methoxyethyl group, a butoxyethyl group, an ethoxyethoxyethyl group and the like.

In the general formula (4), X⁻ representing an ion for neutralization of an electric charge represents an organic or inorganic anion. In the case where X⁻ is a monovalent anion, one mole is necessary and in the case where X⁻ is a divalent anion, a half mole is necessary. Examples thereof include: organic ions such as a methanesulfonate ion, a benzenesulfonate ion, a p-toluenesulfonate ion, a methylsulfate ion, an ethylsulfate ion, a propylsulfate ion, a tetrafluoroborate ion, a tetraphenylborate ion, a hexafluorophosphate ion, a benzenesulfinate ion, an acetate ion, a lactate ion, a trifluoroacetate ion, a propionacetate ion, a benzoate ion, an oxalate ion, a succinate ion, a malonate ion, a oleate ion, a stearate ion, a citrate ion, a dihydrophosphate ion, a tin pentachlorate ion, a chlorosulfonate ion, a fluorosulfonate ion, a trifluoromethanesulfonate ion, a hexafluoroarsenate ion, a naphthalenemonosulfonate ion, a naphthalenedisulfonate ion, a chlorobenzesulfonate ion, a nitrobenzenesulfonate ion, a dodecylbenzenesulfonate ion, a benzenesulfonate ion, an ethanesulfonate ion, a trifluoromethanesulfonate ion, a tetraphenylborate ion, and a butyltriphenylborate; and inorganic ions such as halogen ions including a fluorine ion, a chlorine ion, a bromine ion and an iodine ion, a thiocyanate ion; a perchlorate ion, a periodate ion, a tetrafluoroborate ion, a hexafluorophosphate ion, a molybdate ion, a tungstate ion, a titanate ion, a vanadata ion, a phosphate ion, a borate ion, a nitrate ion, a hexafluoroantimonate ion, a molybdate ion, a tungstate ion, a titanate ion, and a zirconate ion. Among these inorganic ions, ones having a high decomposition temperature are preferable. More preferable are a tetrafluoroborate ion, a hexafluorophosphate ion, a hexafluoroantimonate ion,a nitate ion, an iodine ion and the like.

Next, preferable examples of the compound represented by the general formula (4), although not particularly limited, include specific compounds described below.

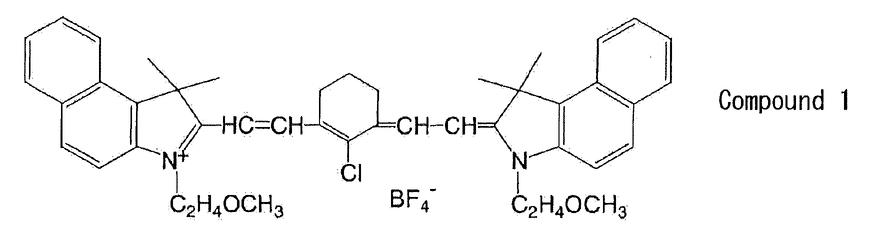

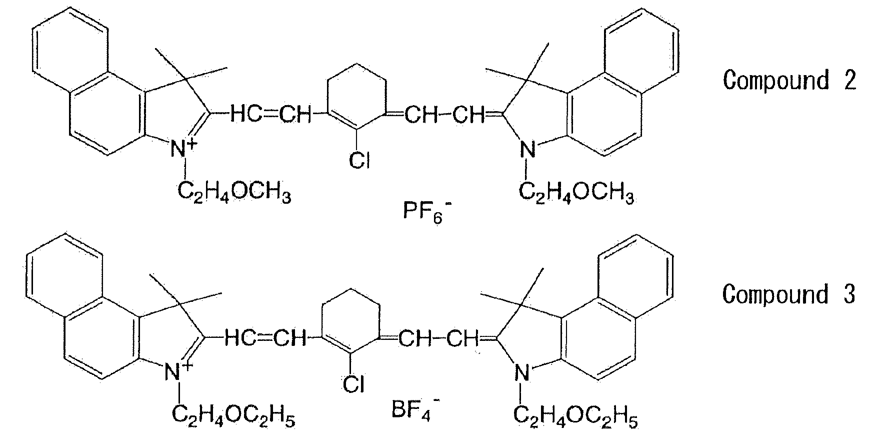

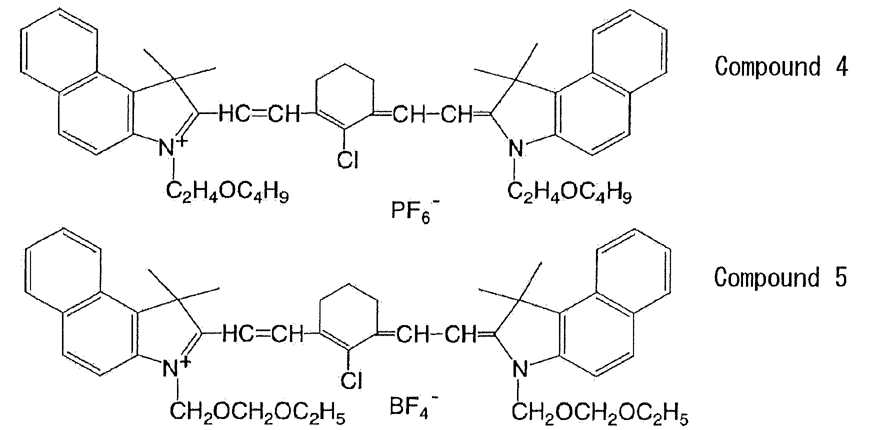

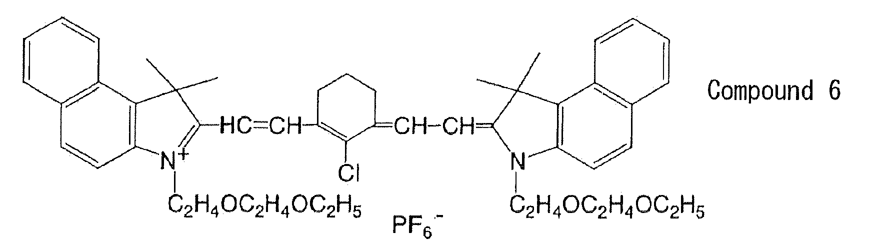

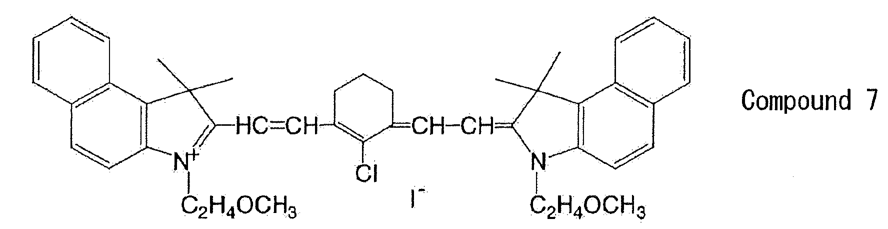

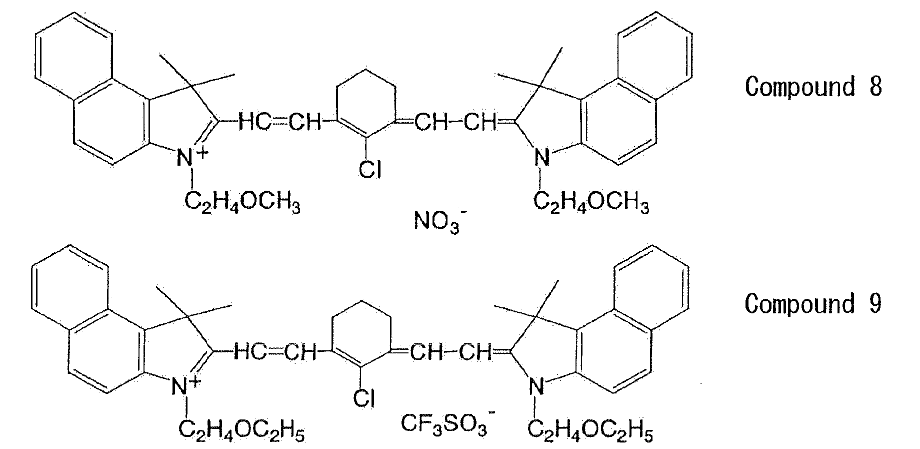

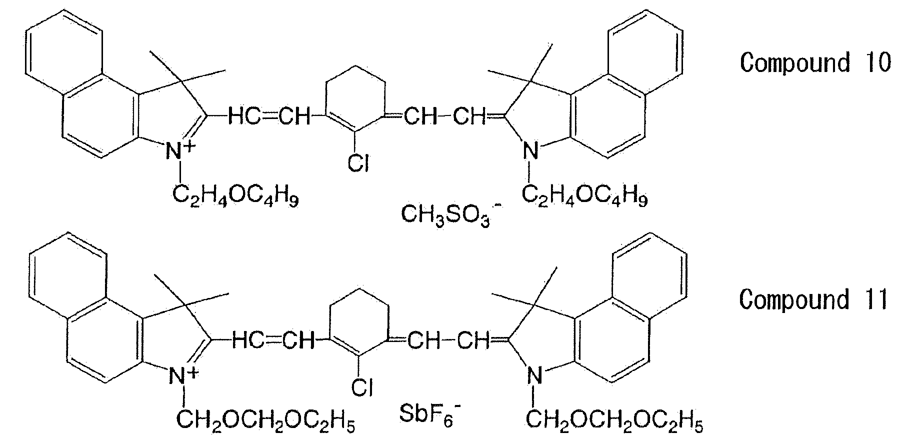

The compound of present invention represented by the general formula (4) can be obtained, for example, by the following method in accordance with a method described in Japanese Patent Publication (KOKOKU) No. 5-37119.

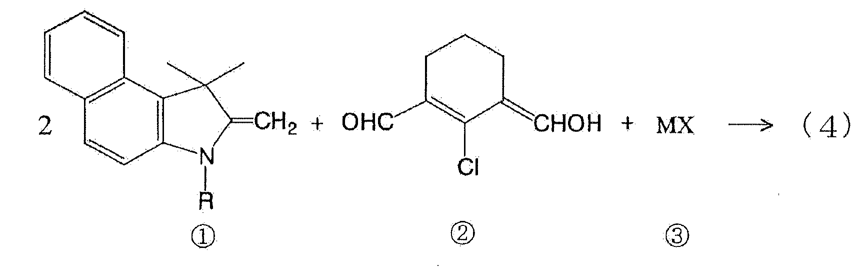

In the above synthesis route, R and X represent the above-described meanings, and M represents a metal, for example, an alkali metal ion or an alkaline earth metal ion for neutralizing an electric charge.

The reaction method will be described in detail. In a solvent such as methanol, ethanol, isopropyl alcohol, glycol, and dimethylformamide, or acetic acid and acetic anhydride, raw material 1, raw material 2, and raw material 3 were charged and the reaction is allowed to continue usually for 1 to 12 hours at 50°C to 140°C to obtain a compound of interest. In this reaction, a base catalyst such as piperazine, piperidine and sodium acetate may be added as a catalyst depending on the case. Alternatively, raw materials 1 and 2 are reacted with each other by the above method to synthesize pigment skeleton, and then may be reacted with raw material 3.

The compound of the present invention has high heat resistance, an absorbing ability in the near-infrared region, particularly 800 to 1100 nm, a high Molar extinction coefficient, and extremely low absorbance in a visible region (generally 400 to 750 nm). Therefore, the compound of the formula (4) according to the present invention is useful as a near-infrared absorber, and further useful for a toner for noncontact thermal fixing type such as flash fixing type.

The toner of the present invention is composed of at least a binder resin, a compound of formula (4) according to the present invention, and a coloring agent. A toner particle can be produced by conventionally and publicly known methods such as a melting, kneading and crushing method or wet granulation method, using well-known binder resins, various coloring agents or the like, which are conventionally and commonly used. The detailed explanation thereof is described in Japanese Patent Application Laying Open (KOKAI) No. 63-161460, Japanese Patent Publication (KOKOKU) No. 7-23965, Japanese Patent Application Laying Open (KOKAI) No. 2000-214626, Japanese Patent Application Laying Open (KOKAI) No. 2000-214628, Japanese Patent Application Laying Open (KOKAI) No. 2002-156779 and the like.

The compound of the present invention has features, that is, high heat resistance, a high molar extinction coefficient in the near-infrared region, in particular 800 to 1100 nm, and extremely low absorbance in a visible region (generally 400 to 750 nm). Thus, when the compound is used for a toner for a noncontact thermal fixing method such as flash fixing method, flash light can be efficiently converted into heat, thereby indicating that fixability becomes high. Further, since the amount of an infrared absorber to be used for a toner can be decreased, it is indicated that the color tone changes of a color toner caused by the infrared absorber are reduced and color images with excellent color reproducibility as well as that toner production cost is reduced.

### EXAMPLES

The present invention will hereinafter be described in more detail by referring to Examples, although it is not limited to these Examples. In the Examples, "parts" means "parts by weight" unless otherwise specified.

### Example 1

2.7 parts of 4,5-benzo-1-(2-methoxyethyl)-3,3-dimethyl-2-methyleneindoline and 0.8 parts of 2-chloro-1-formyl-3-hydroxymethylenecyclohexene were boiled in 4.0 parts of acetic anhydride for 1 hour under reflux, and then cooled to room temperature. Thereafter, the reaction solution was subjected to suction filtration to remove insoluble impurities. Next, the reaction solution was injected into 4.0 parts of water with 0.5 parts of sodium tetrafluoroborate dissolved therein, and precipitated crystals were filtrated by suction and recrystallized with 2.0 parts of DMF. The resultant crystals were washed with 2.0 parts of methanol, and dried, thereby obtaining 2.5 parts of the above-mentioned compound 1. The thus obtained compound 1 had the following physical properties. It should be noted that a value of thermogravimetry-differential thermal analysis (TG-DTA) is shown as a decomposition temperature.
Maximum absorption wavelength: 820 nm (in methanol)
Molar extinction coefficient: 270,000 (in methanol)
Decomposition temperature: 227°C (TG-DTA)

### Example 2

2.7 parts of the above-mentioned compound 2 was obtained in the same manner as in Example 1, except that 0.7 parts of sodium hexafluorophosphate was used instead of sodium tetrafluoroborate. The obtained compound 2 had the following physical properties.
Maximum absorption wavelength: 820 nm (in methanol)
Molar extinction coefficient: 270,000 (in methanol)
Decomposition temperature: 234°C (TG-DTA)

### Example 3

3.0 parts of the above-mentioned compound 7 was obtained in the same manner as in Example 1, except that 0.9 parts of potassium iodide was used instead of sodium tetrafluoroborate. The obtained compound 7 had the following physical properties.
Maximum absorption wavelength: 820 nm (in methanol)
Molar extinction coefficient: 270,000 (in methanol)
Decomposition temperature: 214°C (TG-DTA)

### Example 4

2.7 parts of the above-mentioned compound 5 was obtained in the same manner as in Example 1, except that 3.1 parts of 4,5-benzo-1-(2-(2-ethoxy)ethoxyethyl)-3,3-dimethyl-2-methyleneindoline was used instead of 4,5-benzo-1-(2-methoxyethyl)-3,3-dimethyl-2-methyleneindo line. The obtained compound 5 had the following physical properties.
Maximum absorption wavelength: 820 nm (in methanol)
Molar extinction coefficient: 272,000 (in methanol)
Decomposition temperature: 225°C (TG-DTA)

### Example 5

2. 6 parts of the above-mentioned compound 4 was obtained in the same manner as in Example 1, except that 2.9 parts of 4,5-benzo-1-(2-butoxyethyl)-3,3-dimethyl-2-methyleneindoline and 0.7 parts of sodium hexafluorophosphate were used instead of 4,5-benzo-1-(2-methoxyethyl)-3,3-dimethyl-2-methyleneindoline and sodium tetrafluoroborate, respectively. The obtained compound 4 had the following physical properties.
Maximum absorption wavelength: 822 nm (in methanol)
Molar extinction coefficient: 274,000 (in methanol)
Decomposition temperature: 233°C (TG-DTA)

### Example 6

2.9 parts of the above-mentioned compound 11 was obtained in the same manner as in Example 1, except that 3.1 parts of 4,5-benzo-1-(2-(2-ethoxy)ethoxyethyl)-3,3-dimethyl-2-meth yleneindoline and 1.1 parts of sodium hexafluoroantimonate were used instead of 4,5-benzo-1-(2-methoxyethyl)-3,3-dimethyl-2-methyleneindoline and sodium tetrafluoroborate, respectively. The obtained compound 11 had the following physical properties.
Maximum absorption wavelength: 820 nm (in methanol)
Molar extinction coefficient: 272,000 (in methanol)
Decomposition temperature: 226°C (TG-DTA)

### Example 7

2.3 parts of the above-mentioned compound 8 was obtained in the same manner as in Example 1, except that 0.5 parts of sodium nitrate was used instead of sodium tetrafluoroborate. The obtained compound 8 had the following physical properties.
Maximum absorption wavelength: 820 nm (in methanol)
Molar extinction coefficient: 270,000 (in methanol)
Decomposition temperature: 212°C (TG-DTA)

### Comparative Example 1

2.5 parts of the following Comparative Example Compound 1 (a compound described in Japanese Patent Publication (KOKOKU) No. 5-37119) was obtained in the same manner as in Example 1, except that 2.2 parts of 1-(2-methoxyethyl)-3,3-dimethyl-2-methyleneindoline was used instead of 4,5-benzo-1-(2-methoxyethyl)-3,3-dimethyl-2-methyleneindoline. The obtained Comparative Example Compound 1 had the following physical properties.
Maximum absorption wavelength: 780 nm (in methanol)
Molar extinction coefficient: 249,000 (in methanol)
Decomposition temperature: 210°C (TG-DTA)

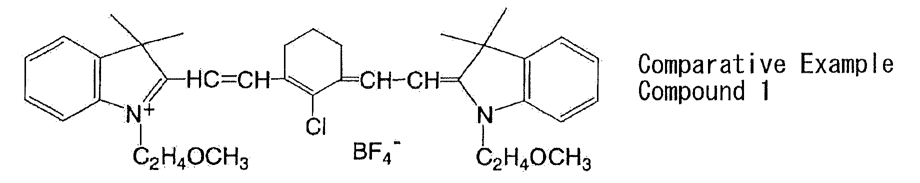

### Comparative Example 2

2.2 parts of the following Comparative Example Compound 2 (a compound described in Japanese Patent Application Laying Open (KOKAI) No. 2001-105756) was obtained in the same manner as in Example 1, except that 2.5 parts of 4,5-benzo-1,3,3-trimethyl-2-methyleneindoline was used instead of 4,5-benzo-1-(2-methoxyethyl)-3,3-dimethyl-2-methyleneindoline. The obtained Comparative Example Compound 2 had the following physical properties.
Maximum absorption wavelength: 814 nm (in methanol)
Molar extinction coefficient: 248,000 (in methanol)
Decomposition temperature: 242°C (TG-DTA)

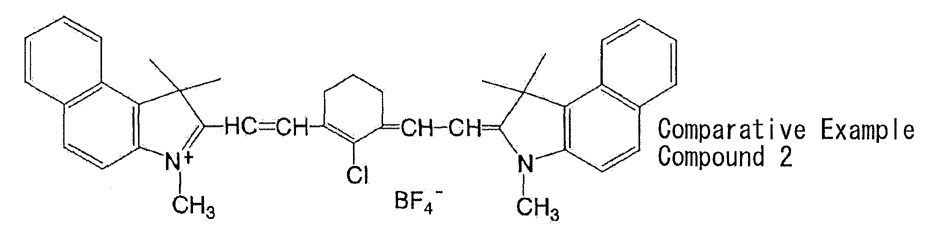

### Comparative Example 3

2.0 parts of 4,4'-bis(diethylaminophenyl)ethylene and 1. 0 part of ortho triethyl formate were charged into 4.0 parts of acetic anhydride, and 0.4 parts of 42% fluoroboric acid was added dropwise thereto. The resultant mixture was boiled for 1 hour under reflux, and then cooled to room temperature. Thereafter, the reaction solution was subjected to suction filtration to remove insoluble impurities. The entire amount of the obtained filtrate was poured into 4.0 parts of water, and precipitated crystals were filtrated by suction, washed with 2.0 parts of methanol, and dried, thereby obtaining 2.0 parts of the following Comparative Example Compound 3 (a compound described in Japanese Patent Application Laying Open (KOKAI) No. 58-219090). The obtained Comparative Example Compound 3 had the following physical properties.
Maximum absorption wavelength: 820 nm (in methanol)
Molar extinction coefficient: 183,000 (in methanol)
Decomposition temperature: 156°C (TG-DTA)

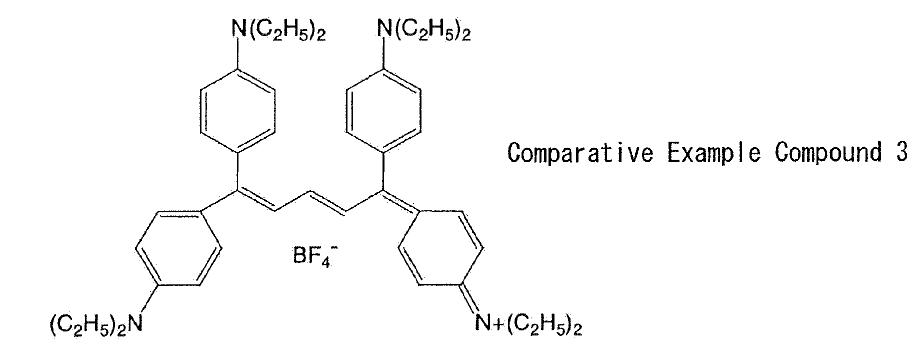

### Comparative Example 4

2.7 parts of the following compound was obtained in the same manner as in Example 1, except that 0.9 parts of sodium p-toluenesulfonate was used instead of sodium tetrafluoroborate. The following compound thus obtained had the following physical properties.
Maximum absorption wavelength: 820 nm (in methanol)
Molar extinction coefficient: 270,000 (in methanol)
Decomposition temperature: 215°C (TG-DTA)

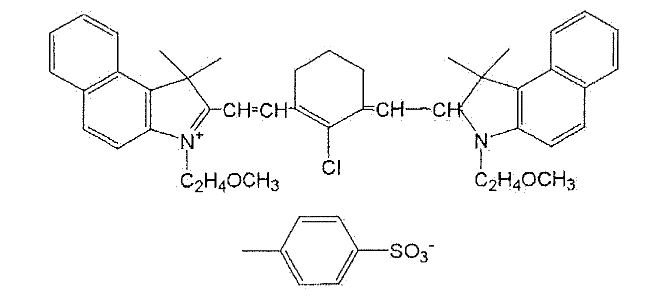

Comparative Example 4 had 11% larger weight of organic anions than a cation thereof in Example 1. That is, absorbance per weight decreases by slightly over 10%, and the decomposition temperature also decreases as a common organic aniondecreases. Inproducing a toner of the present invention, when it is heated and kneaded with a resin, it is considered that this Comparative Example 4 regrettably has no advantages compared with an inorganic anion.

### Comparative Examples 5 and 6

2.4 parts of the following compound was obtained in the same manner as in Example 1, except that 0.6 parts of potassium chlorate was used instead of sodium tetrafluoroborate. The following compound thus obtained had the following physical properties.
Maximum absorption wavelength: 820 nm (in methanol)
Molar extinction coefficient: 270,000 (in methanol)
Decomposition temperature (chlorate ion): 158°C (TG-DTA)

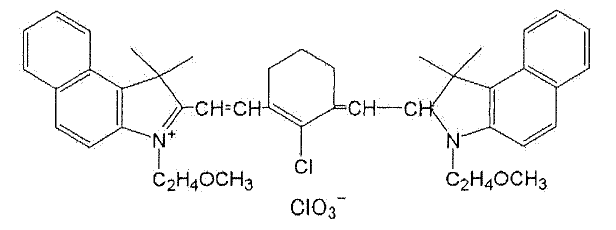

In addition, 2.4 parts of the following compound was obtained in the same manner as in Example 1, except that 0.6 parts of sodium perchlorate was used instead of sodium tetrafluoroborate. The following compound thus obtained had the following physical properties.
Maximum absorption wavelength: 820 nm (in methanol)
Molar extinction coefficient: 270,000 (in methanol)
Decomposition temperature (perchlorateion): 230°C (TG-DTA)

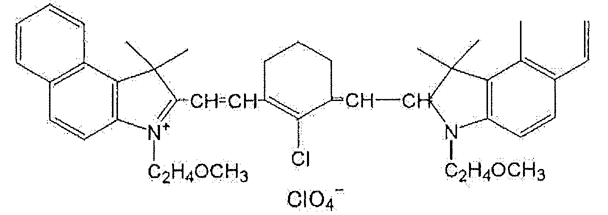

It is well known that chlorate and perchlorate ion forms are easily decomposed at a decomposition temperature. In the compound of the present invention, particularly a chlorate ion form has a remarkably low decomposition temperature. At that temperature, the weight decreased at once by 70% or more and radical decomposition was observed. In addition to dangers in compound production, these Comparative Examples are considered to have no advantage compared with other anions when heated and kneaded with a resin in producing the toner of the present invention.

Table 1 described below shows comparison in absorbance at 600, 650 and 700 nm in methanol of compounds of Examples 1 to 7 with Comparative Example Compounds 1 to 3.

**Table 1**

| (comparison of spectral characteristics in 3 mg/L methanol solution) | | | |
|---|---|---|---|
| | 600nm | 650nm | 700nm |
| Example 1 | 0.014 | 0.052 | 0.147 |
| Example 2 | 0.014 | 0.051 | 0.144 |
| Example 3 | 0.013 | 0.048 | 0.134 |
| Example 4 | 0.012 | 0.048 | 0.136 |
| Example 5 | 0.016 | 0.047 | 0.126 |
| Example 6 | 0.014 | 0.045 | 0.119 |
| Example 7 | 0.014 | 0.053 | 0.148 |
| (hereinafter described with a ratio (%) to Example 1) | | | |
| Comparative Example 1 | 0.024 | 0.089 | 0.274 |
| | (170%) | (170%) | (190%) |
| Comparative Example 2 | 0.018 | 0.071 | 0.199 |
| | (130%) | (140%) | (140%) |
| Comparative Example 3 | 0.213 | 0.262 | 0.164 |
| | (1520%) | (500%) | (120%) |

The compound of the present invention is characterized by having a sharp absorption in the infrared region, a high extinction coefficient, and a high decomposition temperature.

Further, according to Table 1, the compounds of the present invention has an extremely low absorption compared with the Comparative Example Compounds in the visible region, in particular the visible region close to the near infrared region, 600 nm to 700 nm. This difference indicates that the compounds of the present invention has much lower absorption in the visible region than the compounds for comparison and exert very small impact to the color tones of color toners. In addition, since the indolenine compound of the present invention has a high molar extinction coefficient, a toner can be obtained which has excellent fixing in noncontact thermal fixing method such as flash fixing method.

## Claims

1. An indolenine compound represented by the following general formula (1): 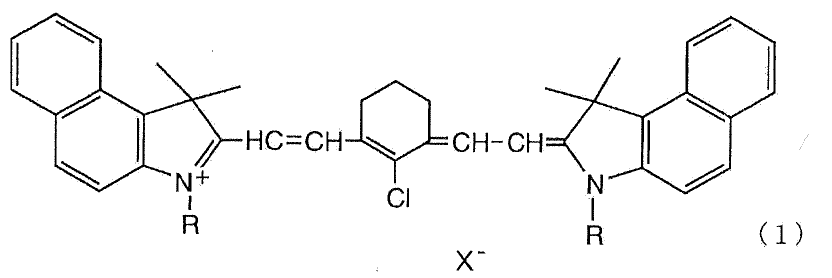 wherein R represents a group represented by the following general formula (2):
-((CH₂)_{b}O)ₐ(CH₂)_{c}OR₁ (2)
wherein a, b and c each represent an integer: a is 1 or 0; b and c each independently represent 1 or 2; and R₁ represents an alkyl group having 4 or less carbon atoms; and
X⁻ represents BF₄⁻, PF₆⁻, I⁻ or NO₃⁻.

2. The indolenine compound according to claim 1, wherein R represents the following general formula (3):
-(CH₂)_{c}OR₁ (3)
wherein c represents 1 or 2, and R₁ represents an alkyl group having 4 or less carbon atoms.

3. An infrared absorber containing as an active ingredient the indolenine compound according to claim 1 or 2.

4. A toner containing an indolenine compound represented by the following general formula (4): 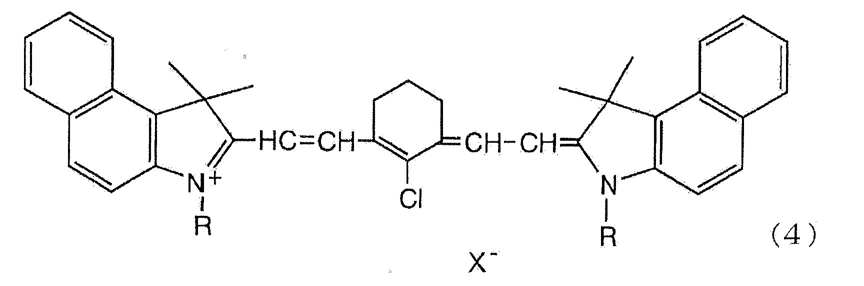 wherein R represents a group represented by the following general formula (5):
- ((CH₂)_{b}O)ₐ(CH₂)_{c}OR₁ (5)
wherein a, b and c each represent an integer: a is 1 or 0; b and c each independently represent 1 or 2; and R₁ represents an alkyl group having 4 or less carbon atoms; and
X- represents an ion for neutralization of an electric charge.

5. The toner according to claim 4, wherein R represents the following general formula (6):
- (CH₂)_{c}OR₁ (6)
wherein c represents 1 or 2, and R₁ represents an alkyl group having 4 or less carbon atoms.

6. The toner according to claim 4 or 5, wherein X⁻ represents BF₄⁻, PF₆⁻, I⁻, NO₃⁻ or SbF₆⁻
